# EUROPEAN PATENT SPECIFICATION

(11) **EP 1 204 381 B1**
(45) Date of publication and mention of the grant of the patent: **23.11.2005**
(21) Application number: 00948223.3
(22) Date of filing: 28.07.2000
(51) Int. Cl.: A61B 17/34

(54) **A SURGICAL ACCESS DEVICE**
CHIRURGISCHE ZUGANGSVORRICHTUNG
DISPOSITIF CHIRURGICAL

(30) Priority: 30.07.1999 IE 990659
(43) Date of publication of application: 15.05.2002
(73) Proprietor: Gaya Limited, Dublin 18 (IE)
(72) Inventor: CALDWELL, Martin, Tromode, Isle of Man (GB); CUMMINS, Christopher, Naas, Co. Kildare (IE)
(74) Representative: McCarthy, Denis Alexis
(86) International application number: PCT/IE2000/000093
(87) International publication number: WO 2001/008563

(56) References cited:
- EP-A- 0 487 175
- EP-A- 0 542 428
- WO-A-95/11050
- WO-A-96/10432
- WO-A-97/31578
- GB-A- 2 103 936
- US-A- 4 573 576
- US-A- 5 279 575
- US-A- 5 514 133
- US-A- 5 658 272
- US-A- 5 658 306
- US-A- 5 817 062
- US-A- 5 830 191
- US-A- 5 882 344

## Description

The present invention relates to a surgical device and more particularly to a trocar and cannula for use in minimally invasive surgery of the type using patient pneumoperitoneum and an access port.

Minimally invasive surgery of this type is carried out having introduced gas into a patient's body cavity through an incision and having sealed the incision with an access port. The access port enables hand assisted laproscopic surgery (HALS) or instrument assisted surgery to be performed. When the surgery requires the use of a second set of instruments, a trocar is often used to introduce a cannula as an alternative to a second incision and access port. A trocar is a surgical device designed to provide a small conduit known as a cannula through tissue to allow instruments such as an endoscope to be inserted into the body cavity.

The uses of trocar and cannula devices have a number of problems generally. Previously known cannulas are prone to allowing gas to escape from the body cavity when in position. The gas may escape through the cannula while a surgical instrument is in use or from around the incision. The provision of a suitable seal is essential in maintaining the correct pressure of gas within the body cavity, however, it is also essential to ensure that the seal is provided in a manner which does not limit a surgeon's mobility or cause unnecessary trauma. This can prove particularly problematic where the device is to be inserted in a narrow portion of the body cavity. Existing cannulas are also prone to being ejected from the body cavity due to gas pressure or instrument removal force, which is obviously unacceptable.

Additionally, fixing the cannula, once successfully in position, has presented difficulties. If the fixing arrangement is too large it can limit a surgeon's mobility and if the arrangement extends too far into the cavity there is a risk of unnecessary trauma. Furthermore, the insertion of the trocar often inadvertently causes unacceptable damage to a patient's tissue and internal organs.

European Patent Specification No. EP 0 542 428 discloses a surgical trocar assembly for insertion of a laparoscopic instrument through an inner abdominal wall. A sleeve is provided which can be expanded within the abdominal cavity to abut against the surface of the abdominal wall to hold the sleeve in place, in combination with insulation to electrically insulate the trocar assembly from the patient while the instrument is within the trocar assembly.

United States Patent Specification No. US 5,658,306 discloses a method for locating additional cannulae in surgery on a patient with the use of a guide member having proximal and distal extremities, comprising introducing the distal extremity of the guide member through the skin of the body to a first location in the body. The distal extremity of the guide member is advanced in a direction below the skin to a second location remote from the first location. The distal extremity of the guide member is advanced through the skin of the patient at the second location from within the body to a position outside the body so that the distal extremity is exposed outside the body. A cannula is placed over the distal extremity of the guide member and is advanced through the skin of the patient at the second location utilizing the guide member as a guide. The distal extremity of the guide member is then removed from the cannula.

It is an object of this invention to provide a method and apparatus for inserting and using a cannula, which will overcome the aforementioned problems.

It is a further object of the invention to provide an apparatus for securing and using a cannula, which will overcome the aforementioned problems and which will reduce the risk of secondary seeding of cancerous cells at port sites.

Accordingly, there is provided a surgical device for use in minimally invasive surgery of the type where a body cavity is inflated to be accessible of a surgeon through an access port surrounding an incision in a patient's body, the device being formed to allow insertion of medical equipment and comprising: -
a cannula defining a conduit into the body cavity,
a trocar carried on the cannula and formed for piercing or cutting tissue to position the cannula; and
fixing means for removably securing the cannula in position on the patient during surgery,
characterised in that the trocar is removably mounted on the cannula, with the trocar providing a gas-tight cap for the cannula when the trocar is mounted on the cannula thereby enabling the trocar to be inserted into the body cavity through the access port and to cut or pierce tissue outwardly from within the body cavity put to an operating site, with no escape of gas from the body cavity:

In this way accidental damage to the patient's tissue or organs is prevented as the cutting or piercing point of the trocar is directed away from the patient's body during the fixing procedure.

Ideally, the device is formed for insertion into the body cavity through the access port and for cutting or piercing tissue outwardly from within the body cavity out to an operating site. Thus, the action of the trocar piercing the body cavity wall automatically draws the cannula into position.

Preferably, the trocar is removably mounted on the cannula.

In a particularly preferred embodiment, the trocar and cannula are connected with complementary engageable short threads on the trocar and cannula, such as quarter turn threads.

Since the trocar is formed to provide a gas-tight cap for the cannula when mounted thereon, as the trocar pierces outwardly from the body cavity there is no escape of gas, which would collapse the cavity. This is particularly useful when a single external valve is used.

In one arrangement, the trocar is provided with an integral cutting element. This facilitates the cutting of the body cavity wall to allow introduction of the cannula.

Preferably, the trocar incorporates an extension shoulder, thereby allowing the trocar to define a narrow incision, which is gently extended to ensure that the drawn cannula is firmly lodged in the body cavity wall preventing loss of gas from the cavity.

Preferably, the trocar incorporates guard means for protecting a surgeon's hand from being cut when being introduced into the body cavity or as the device cuts the patients skin thereby preventing injury to the surgeon.

Preferably, the cannula incorporates means for attaching the cannula to an interior of the body cavity when in position.

Ideally, the means for attaching the cannula to an interior of the body cavity is provided by an internal distal ring.

In one arrangement, the internal distal ring and the cannula are integrally formed as a single unit.

Preferably, the cannula incorporates one or more valves, internally or externally of the body cavity, to prevent loss of gas from the body cavity when the cannula is in position.

In one arrangement, the fixing means incorporates an anchor ring formed for releasable engagement with a proximal end of the cannula extending from the body when the cannula is in position in the body cavity. In this way, when the trocar has pierced the body cavity wall from within the cavity the portion of the cannula extending through the wall can be used to secure the cannula in position. This prevents tearing of the body tissue as the cannula is firmly attached to both internal and external walls of the body cavity.

Preferably, the anchor ring incorporates a thread for engaging on the same thread used to secure the trocar prior to installation of the cannula. This reduces the complexity of components and allows the threaded portion on the cannula to serve both functions.

In an alternative arrangement to that described above the valve is incorporated into the anchor ring.

In a particularly preferred embodiment of the invention the device incorporates an external seal and an internal valve, the seal and valve being mounted about opposing ends of the cannula.

Preferably, the cannula is integrally formed with the seal and valve.

In one arrangement, the valve is integrally formed at one end of the cannula and has means for releaseably engaging a seal housing at an opposing end.

Preferably, the seal housing incorporates a diaphragm seal.

In one arrangement, the seal housing defines an extended entry port.

Preferably, the entry port has a conical section.

In a preferred embodiment, the cannula incorporates an insufflation port. In this way the device may also incorporate the functionality required to inflate the body cavity in a single device. This ensures that the operating site is adequately and controllably inflated and that the component count is reduced, thereby, simplifying postoperative procedures.

Ideally, the insufflation port communicates with an insufflation lumen having insufflation ducts communicating between the port and the body cavity.

Preferably, the insufflation lumen is carried on an exterior surface of the cannula.

Preferably, the anchor ring incorporates finger grip means to facilitate engagement with the cannula.

Preferably, the anchor ring and or the distal ring incorporate cushion means to prevent trauma to the body cavity wall and ensure a gas tight seal.

In a particularly preferred arrangement, the device incorporates a detachable security retainer formed for engagement with a surgeon's hand or instrument to prevent loss of the device in the cavity prior to insertion.

According to one aspect of the invention the device preferably incorporates an adjustable pressure release valve. Thus, the surgeon may inflate the body cavity to a desired level and tenting or venting of additional gas added during surgery prevents tearing of tissue surrounding the incisions made. The surgeon is thereby assured of constant body cavity inflation.

In one preferable embodiment the fixing means is provided by:-
an inner seal having an upwardly extending conduit carried on an internal mounting ring; and
an external seal having an downwardly extending conduit carried on an external mounting ring,
the upwardly and downwardly extending conduits being formed for releasable engagement when in position on a patient to define the cannula.

Preferably, the conduits are formed for slidable interengagement.

Ideally, the conduits incorporate a ratchet retainer. This allows one conduit to be slid into position with one conduit being accommodated within the other and the ratchet ensures that the device can accommodate a wide variety of tissue wall widths while also guaranteeing a suitable seal.

In one arrangement, the or each conduit is deformable to facilitate removal by disengaging the ratchet retainer.

Ideally, the, or each mounting ring has an associated pressure absorption and seal enhancement means, provided for example by a sponge.

In a particularly preferred embodiment, the cannula, when in position also incorporates a valve.

Preferably, the mounting rings are pivotally movable about the longitudinal axis. In this way the cannula may be positioned at an angle. This can allow a surgeon to position the cannula in such a way as to direct an inserted piece of equipment to an operating site or to facilitate insertion in a portion of the body cavity where access is limited. Pivotal movement of the mounting rings ensures that seal integrity is maintained.

Preferably, the cannula is collapsible under pressure from one or both mounting rings to secure the cannula in position.

In one arrangement the cannula is provided by a section of pleated tubing carried on the cannula.

The external mounting ring preferably defines an entry funnel having an oversize entry aperture for receiving and facilitating insertion of a piece of medical equipment.

The invention will now be described more particularly with reference to the accompanying drawings, which show, by way of example only, an embodiment of a surgical device in accordance with the invention, in which:
- Fig. 1: is a perspective view of portion of a surgical device in accordance with the invention;
- Fig. 2: is a sectional view of the surgical device of Fig. 1 in position on a patient;
- Fig.3: is a side view of the surgical device in accordance with the invention in a piercing position;
- Fig. 4: is a side view of the device in a partially placed position;
- Fig. 5: is a side view of the device in a pre-locking position;
- Fig. 6: is a side view of the device shown in Figs. 3 to 5 in an in use position;
- Figs. 7 and 8: illustrate operation of a valve for use with the invention;
- Fig. 9: is a sectional view of an alternative embodiment of a surgical device in accordance with the invention in position on a patient;
- Fig. 10: is a top view of portion of the device shown in Fig. 9 in the direction of the arrows X - X;
- Fig. 11: is a sectional view of a further embodiment of a surgical device in accordance with the invention in position on a patient;
- Fig. 12: is a top view of portion of the device shown in Fig. 11 in the direction of the arrows XII - XII;
- Fig. 13: is a sectional view of a surgical device in accordance with the invention;
- Fig. 14: is a sectional view of an alternative embodiment of a surgical device in accordance with the invention in position on a patient;

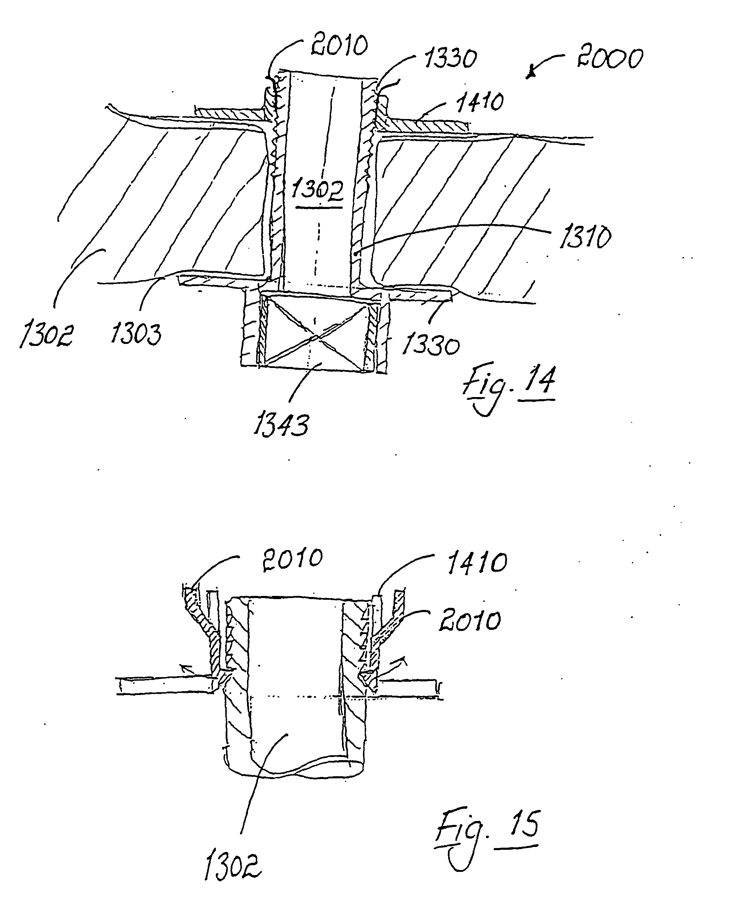

- Fig. 15: is an enlarged sectional view of portion of the surgical device of Fig. 14;
- Fig. 16: is a sectional view of a further alternative embodiment of a surgical device in accordance with the invention in an insertion position;
- Fig. 17: is a sectional view of the surgical device of Fig. 16 in an in use position;
- Fig. 18: is a sectional view of a still further alternative embodiment of a surgical device in accordance with the invention in an insertion position;
- Fig. 19: is a sectional view of the device of Fig. 18 in an in use position;
- Fig. 20: is a sectional view of a still further alternative embodiment of a surgical device in accordance with the invention;
- Fig. 21: is a sectional view of a seal forming part of the invention;
- Fig. 22: is an exploded view of discs forming part of the seal illustrated in Fig. 21;
- Fig. 23: is a sectional view of an alternative seal forming part of the invention; and
- Fig. 24: is a perspective view of the seal illustrated in Fig. 23.

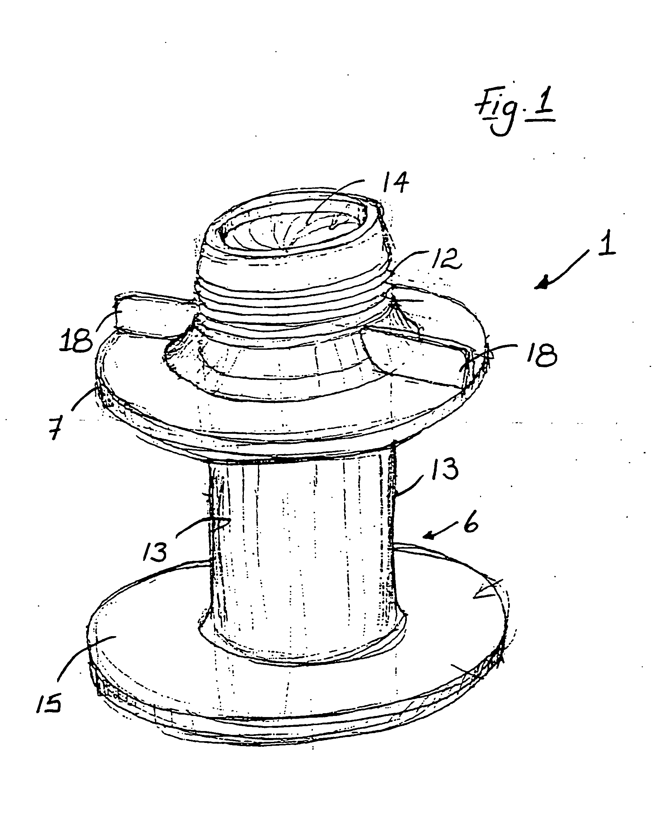

Referring to the drawings, and initially to Figs. 1 to 8 there is illustrated a surgical device according to the invention, indicated generally by the reference numeral 1. The surgical device 1 is formed for use in minimally invasive surgery of the type using an inflated body cavity. The cavity is covered by in this case an abdominal wall 2 having an internal cavity wall 3 and an external wall 4. The internal cavity wall 3 is accessible to a surgeon through an access port (not shown)

In more detail, the device 1 has a trocar 5, a cannula 6 and an anchor ring 7. The trocar 5 has a cutting tip 8, a shoulder portion 9 and an internal thread (not shown) for connecting the trocar 5 to the cannula 6.

The cannula 6 has a threaded portion 12 for connection to the internal thread of the trocar 5. and for extending beyond the external wall 4 when in position. The cannula 6 also has a body section 13 housing a valve 14 to prevent escape of gas from the body cavity when in position and an integrally formed distal ring 15 for engaging the internal wall 3.

The anchor ring 7 has an internal threaded portion 17 formed for engagement with the cannula threaded portion 12 and finger grip means 18 for facilitating this engagement.

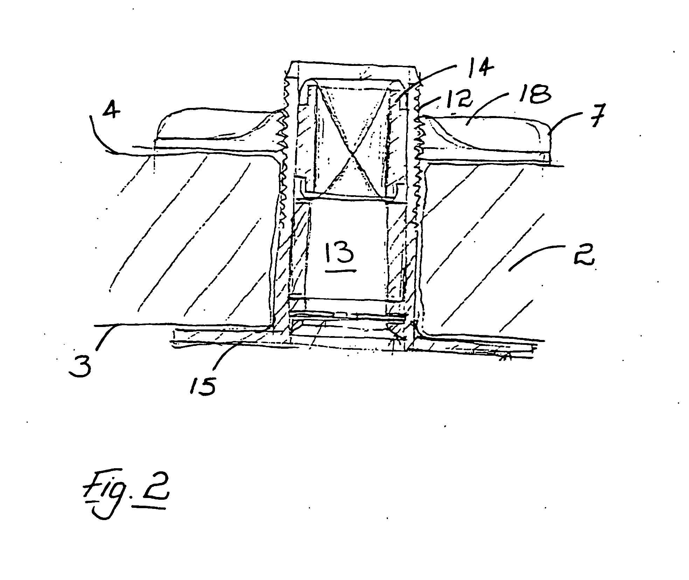

In use, and referring now in particular to Figs. 3 to 6 the device 1 is inserted into the body cavity. The trocar 5 is moved into a piercing position as shown in Fig.3 with cutting tip 8 in contact with the inner wall 3. The cutting tip 8 is then pressed through the abdominal wall 2 until the tip 8 and shoulder portion 9 extends beyond the external wall 4. When the distal ring 15 comes into contact with the internal wall 3 and the body section 13 is in the abdominal wall 2 the trocar 5 is unscrewed from the threaded portion 12 of the cannula 6. (See Fig. 4.)

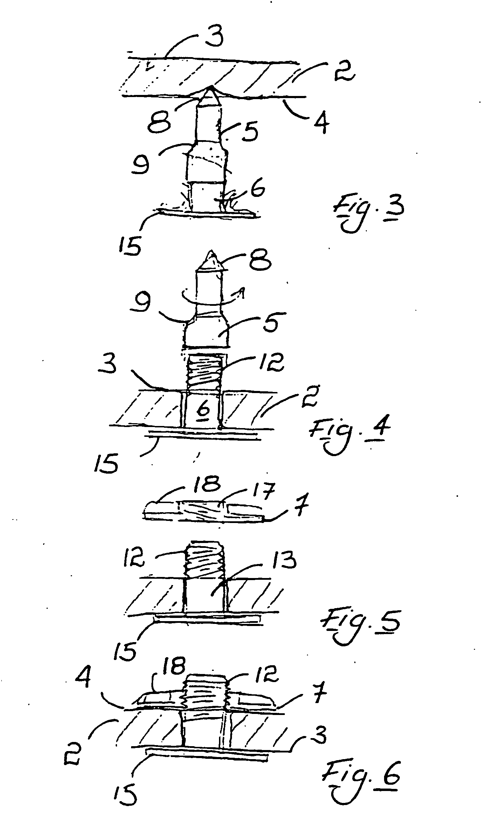

The anchor ring 7 is then ready for engagement as shown in Fig. 5. The finger grip means 18 are used to engage the threaded portion 17 with the threaded portion 12 of the cannula 6 until the anchor ring 7 is firmly pressed against the external wall 4. (See Figs. 2 and 6)

The invention provides a simple device which prevents accidental damage to the patients tissue or organs by ensuring that the cutting or piercing point of the trocar is directed away from the patient's body during insertion. This is achieved in a way that ensures that the cannula is automatically drawn into position.

It will be understood that the cutting or piercing method may optionally include the use of an energy source such as a heater or spring-loaded mechanism for executing the cut using a minimum of force.

To avoid unnecessarily obscuring the present invention, detailed description of the valve has not been included, as it will be apparent to those stalled in the art that a wide variety of valves may be successfully employed. One such possibility is a twist valve, but any suitable valve may be used.

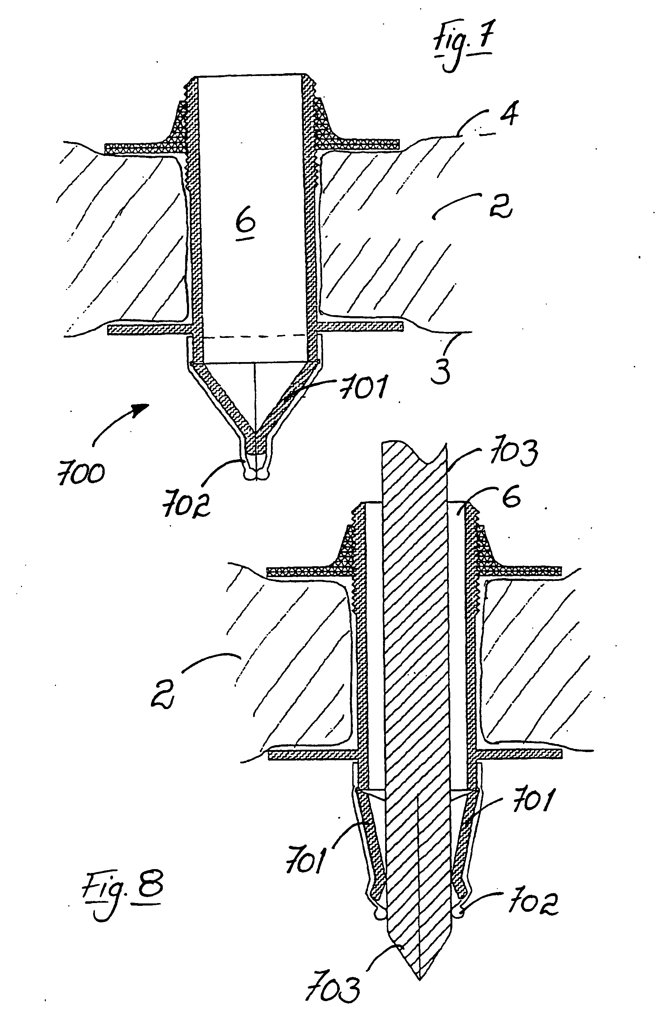

Referring now to Figs. 7 and 8 there is illustrated one example of a valve for use with the device indicated generally by the reference numeral 700 in which parts illustrated in Figs.1 to 6 are indicated by the same numerals generally. With the device in position the valve 700 is located within the body cavity. A pair of doors 701 hingedly connected to the cannula 6 provides the valve. The doors 701 are biased into a closed position by a rubber cover 702. When a surgical implement 703 is inserted into the cannula (See Fig. 8) the doors 701 are forced outwardly against the cover 702. The cover 702 seals around the surgical implement 703 preventing escape of air from the cavity and as the implement is removed they also act to close the doors 701.

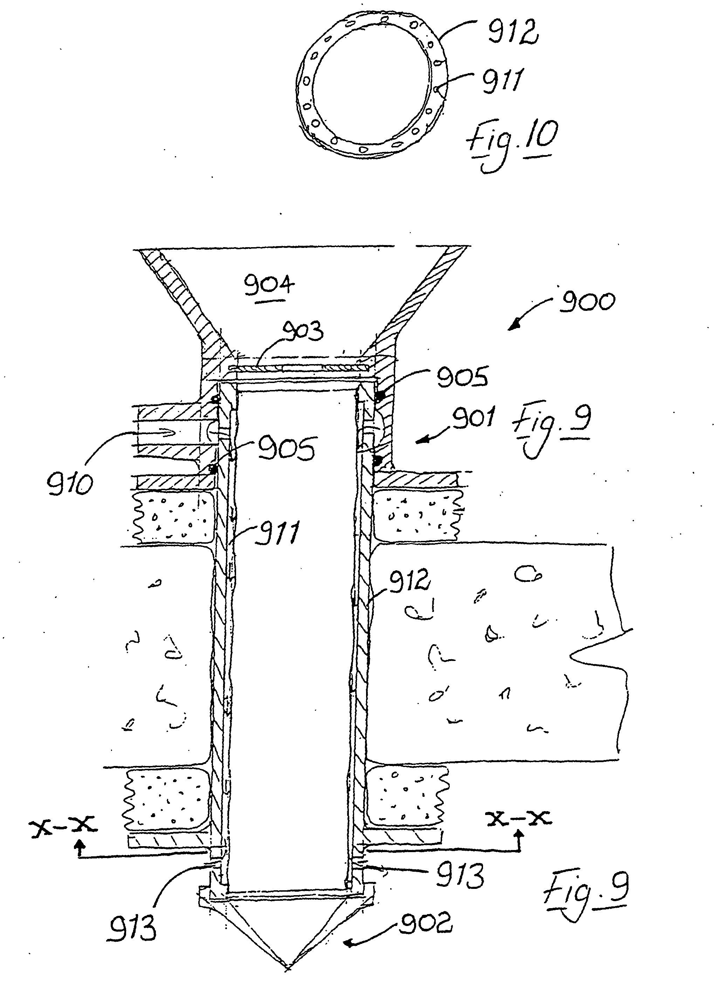

Referring now to Figs. 9 and 10 there is illustrated a further embodiment of a surgical device indicated generally by the reference numeral 900 in which parts similar to those described with reference to Figs. 1 to 8 are identified by the same numerals generally.

In this embodiment, the device 900 incorporates a releaseably attachable external seal housing 901 and an internal one way valve 902 mounted about opposing ends of the cannula 6. The housing 901 is located on the cannula 6 when in position on the patient using O rings 905. The housing 901 incorporates a diaphragm seal 903 and defines an outwardly directed extended entry port indicated at 904. The entry port 904 has a conical section and is so formed to facilitate insertion of a surgical device by directing the device to the cannula 6. The housing 901 also defines an insufflation port 910 for receiving insufflation gases. These gasses are routed to the body cavity to ensure adequate and controlled inflation through insufflation ducts 911 carried in an insufflation lumen 912. The lumen 912 is carried on an exterior surface of the cannula 6 and terminates at the one way valve 903 with an insufflation outlet 913.

In use, the device 900 is positioned on the patient substantially as described with reference to Figs. 1 to 8. When the cannula 6 is in position the housing 901 is fixed in position using the O rings 905. The body cavity may then be inflated. A gas source (not shown) is connected with a pipe to the insufflation port 910. The gas is routed to the body cavity through the insufflation ducts 911 of the insufflation lumen 912 and out into the cavity through the insufflation outlet 913.

Once inflated instruments are passed through the diaphragm seal 903 to enter the cannula 6 having first been introduced and guided into position by a tapering internal surface of the entry port 904. Instruments are then passed down the cannula 6 and into the inflated body cavity through the one way valve 902.

It will be understood that a cannula sheath separated from the cannula body and running along the cannula exterior may equally provide the insufflation lumen.

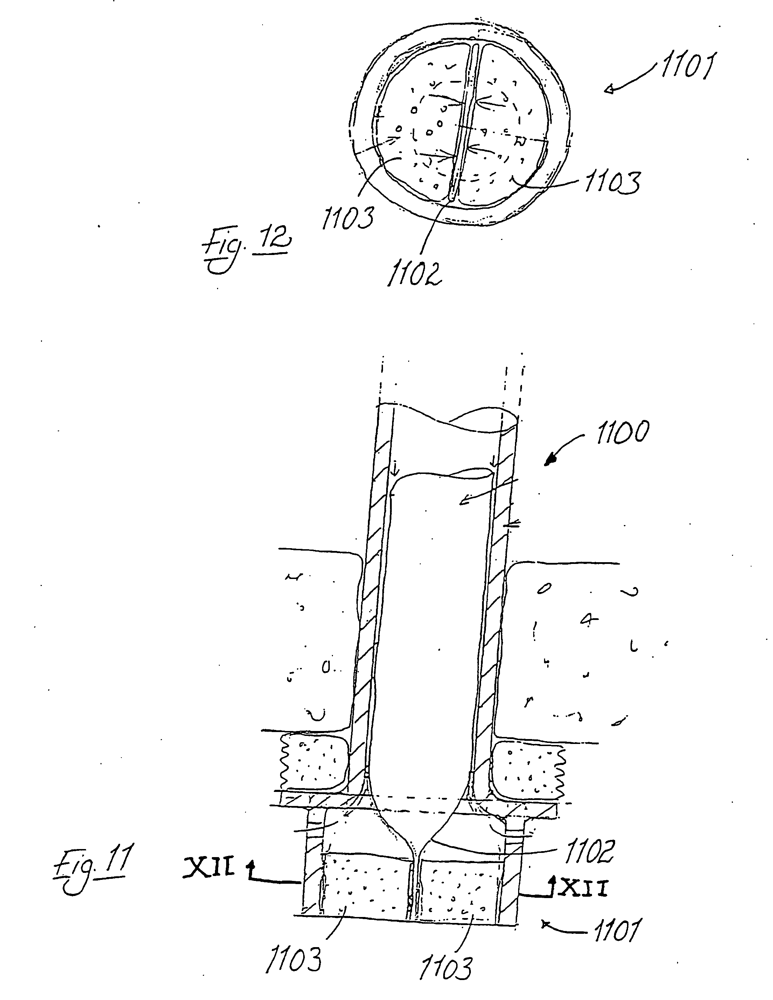

Referring now to Figs. 11 and 12 there is illustrated a further embodiment of a surgical device indicated generally by the reference numeral 1100 in which parts similar to those described with reference to Figs. 1 to 10 are identified by the same numerals generally.

In this embodiment, the cannula 6 terminates in the body cavity with a one way valve 1101. Compressing a thin sleeve 1102 between two semi-circular, flexible foam pieces 1103 forms the valve 1101. The valve allows for the insertion of surgical implements and prevents the escape of gas from the body cavity.

It will be noted that while threaded engagement of the component parts of the device is described throughout any suitable method of engagement is acceptable.

It will also be noted that the distal ring and anchor ring may be suitably coated with airtight, antiseptic and or shock absorbent material to prevent trauma to the abdominal wall and loss of gas from the body cavity.

It will be understood that the anchor ring may be extended to incorporate a valve, which allows insertion of instruments thereby reducing the weight of the device.

It will also be understood that the device may incorporate a detachable security retainer formed for engagement with a surgeon's hand or instrument to prevent loss of the device in the cavity prior to insertion.

It will further be understood that the device may incorporate an adjustable pressure release valve. This will allow the surgeon to inflate the body cavity to a desired level and ensure a constant level of body cavity inflation by continuously supplying inflation gas and venting any excess through the pressure release valve. This prevents excessive movement of the cavity wall during the procedure, which can inconvenience the surgeon and cause trauma to the patient

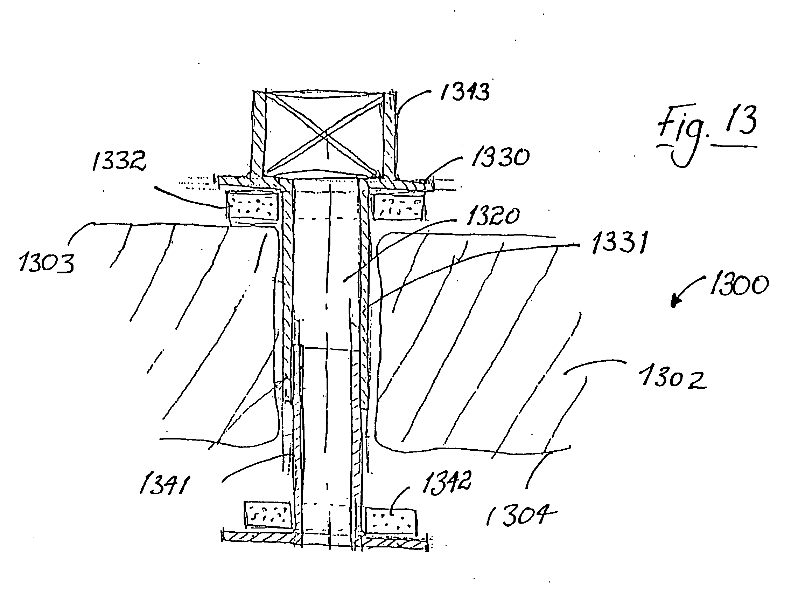

Referring to the drawings again and now to Fig. 13 there is illustrated a surgical device indicated generally by the reference numeral 1300. The surgical device 1300 is formed for use in minimally invasive surgery of the type using an inflated body cavity. The cavity is covered by in this case an abdominal wall 1302 having an internal cavity wall 1303 and an external wall 1304. The internal cavity wall 1303 is accessible to a surgeon through an access port (not shown)

In more detail, the device 1300 has a fixing means provided by an inner sealing ring 1330 and an outer sealing ring 1340. The outer ring 1340 is movable along a longitudinal axis of a cannula 1320 to secure the device 1300 in position on the abdominal wall 1302. The cannula 1320 is defined by the interengagement of an upwardly extending conduit 1331 carried on the ring 1330 and a downwardly extending conduit 1341 carried on the ring 1340. The conduit 1341 has a ratcheted retainer exterior surface portion (not shown) and is dimensioned to slidably engage and ratchet within the conduit 1331 against a ratcheted interior portion thereof (not shown). Once engaged the conduits 1331 and 1341 define the cannula 1320. This form of engagement allows the device 1300 to accommodate a wide variety of walls 1302 while also guaranteeing a suitable seal. The outer ring 1340 has an absorbent sealing sponge 1342. The inner sealing ring 1330 also has a sponge 1332 and a valve 1343 to prevent escape of gas from the cavity.

In use, the device 1300 is inserted into the body cavity by first introducing the inner sealing ring 1330 into the cavity and pushing the conduit 1331 through the wall 1302. Pushing continues until the sponge 1332 comes into contact with the internal cavity wall 1303. The ring 1340 is then positioned over the conduit 1331 with the conduit 1341 extending downwards. The conduit 1341 is then introduced into the conduit 1331 and slideably engaged. Sliding continues until the sponge 1342 presses snugly against the external wall 1304. The device is securely held as the ratcheted portions of the conduits ensure positive contact. The valve 1343 prevents gas from escaping during an operation.

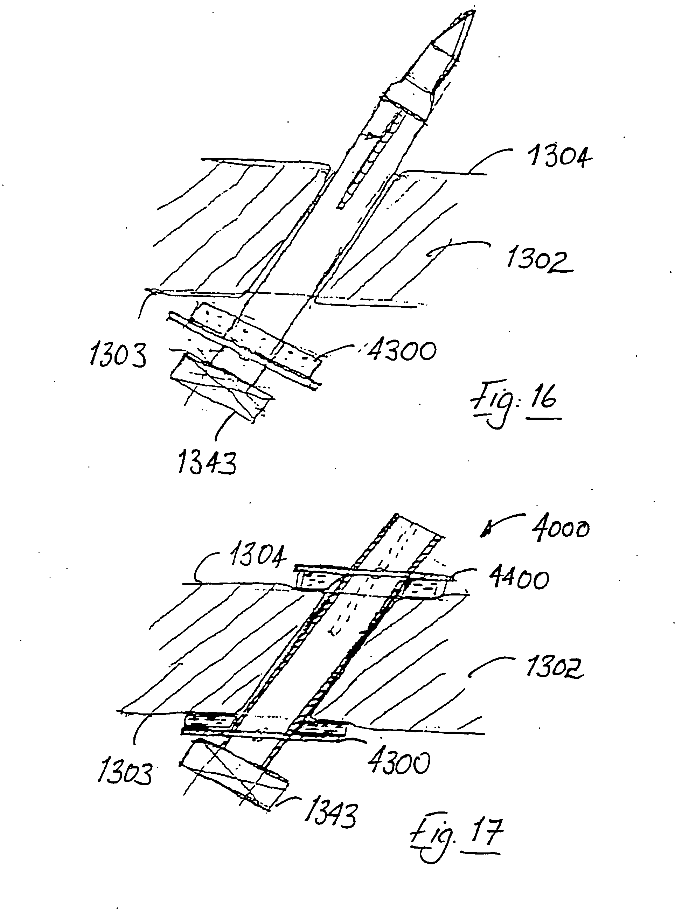

Referring now to Figs. 14 and 17 there is shown an alternative device indicated generally by the reference numeral 2000 in which parts shown in Fig. 13 are identified by the same reference numerals generally. The device 2000 in use operates in a similar fashion but in this embodiment a conduit 1410 is greatly shortened over the conduit 1331 of Fig. 13. A conduit 1310 in this instance has a ratcheted surface 1330 along an exterior portion. A ratchet retainer is provided by the slidable engagement of the surface 1330 against a grip 2010 carried on the ring 1340.

Release of the device 2000 is effected in this instance by squeezing the grip 2010 toward the conduit 1310 to disengage the grip 2010 from the surface 1330.

Referring now to Figs. 16 and 17 there is shown a further embodiment of a device indicated generally by the reference numeral 4000 in which parts shown in Fig. 13 are identified by the same reference numerals generally. The device 4000 has an internal ring 4300 and an external ring 4400 pivotally mounted on a cannula 4200. Engagement of the device 4000 and the rings 4300 and 4400 is similar to that described above. In this embodiment the pivotal movement of the rings about the longitudinal axis of the cannula 4200 allows the cannula to be inserted at an angle. As the ring 4300 approaches the internal wall 1330 the pivotal movement allows the ring 4300 to move to contour the internal wall 1303. Similarly the external ring 4400 moulds the external wall 1304. This movement allows a surgeon to position the cannula in such a way as to direct an inserted piece of equipment to an operating site or to facilitate insertion in a portion of the body cavity where access is limited. Pivotal movement of the mounting rings also ensures that seal integrity is maintained.

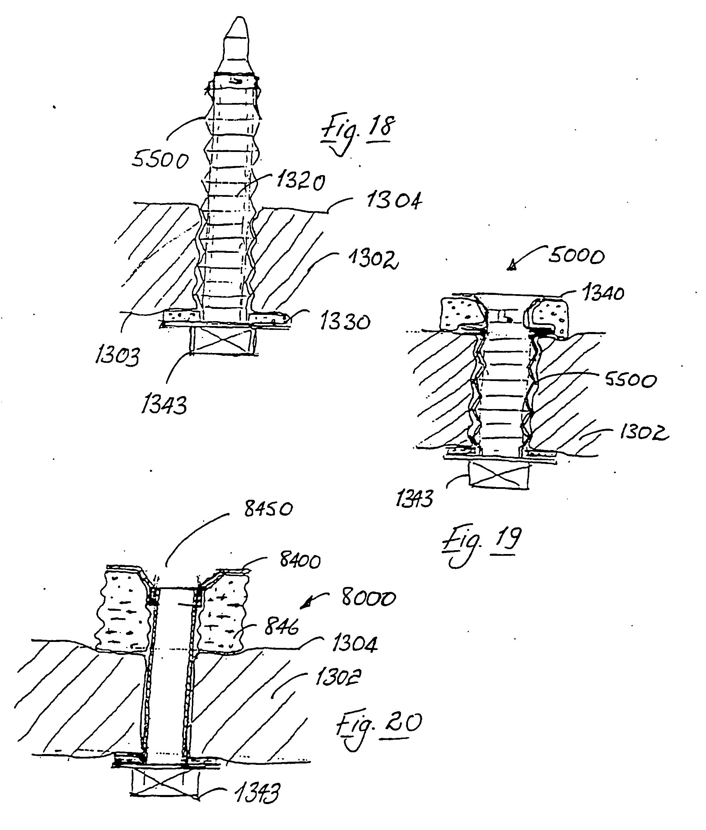

Referring now to Figs. 18 and 19 there is shown a further embodiment of a device indicated generally by the reference numeral 5000 in which parts shown in Fig. 13 are identified by the same reference numerals generally. In this embodiment, the cannula 1320 incorporates a pleated tubing 5500. As the ring 1340 is positioned, the trocar 1350 is removed and the cannula 5500 is collapsed. This collapse causes the cannula 5500 to expand and provides an additional seal around the body incision.

Now referring to Fig. 20 there is shown a further embodiment of a device indicated generally by the reference numeral 8000. In this embodiment the ring 8400 defines a funnel entry 8450 to facilitate insertion of medical instruments. This embodiment also has a foam spacer 8460 which is particular useful when it is desired to extend the cannula.

It is envisaged that the cannula may incorporate a deformable web, such as corrugated tubing, which will deform when in position on a patient to stabilise the cannula and to provide a seal.

It will be understood that the device may include a number of seals and that these seals may operate independently or in combination. For example, the device may include a seal adjacent the outer ring which will engage only when an instrument has been passed into the body cavity and there is a risk of gas escaping from a seal adjacent the inner sealing ring.

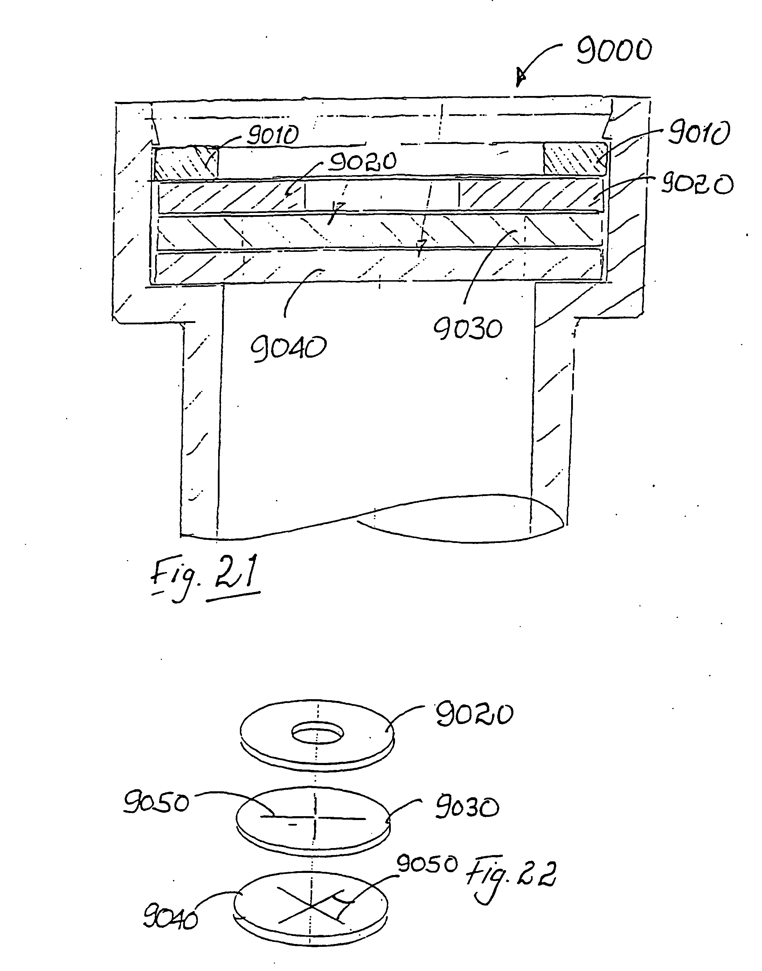

Referring now to Figs. 21 and 22, there is illustrated one such seal forming part of the invention indicated generally by the reference numeral 9000. The seal 9000 has a locking ring 9010, a diaphragm seal 9020 and two slit seals 9030, 9040. The slit seals 9030, 9040 each have four orthogonally arranged slits 9050 radiating from the center and the seals 9030, 9040 are offset by forty five degrees. In use, a seal is provided as an instrument is introduced by the action of pressing the instrument against the slits 9050. When the device is removed, the slits 9050 return to normal resting position offset by forty five degrees from the slit above thereby maintaining an overall seal.

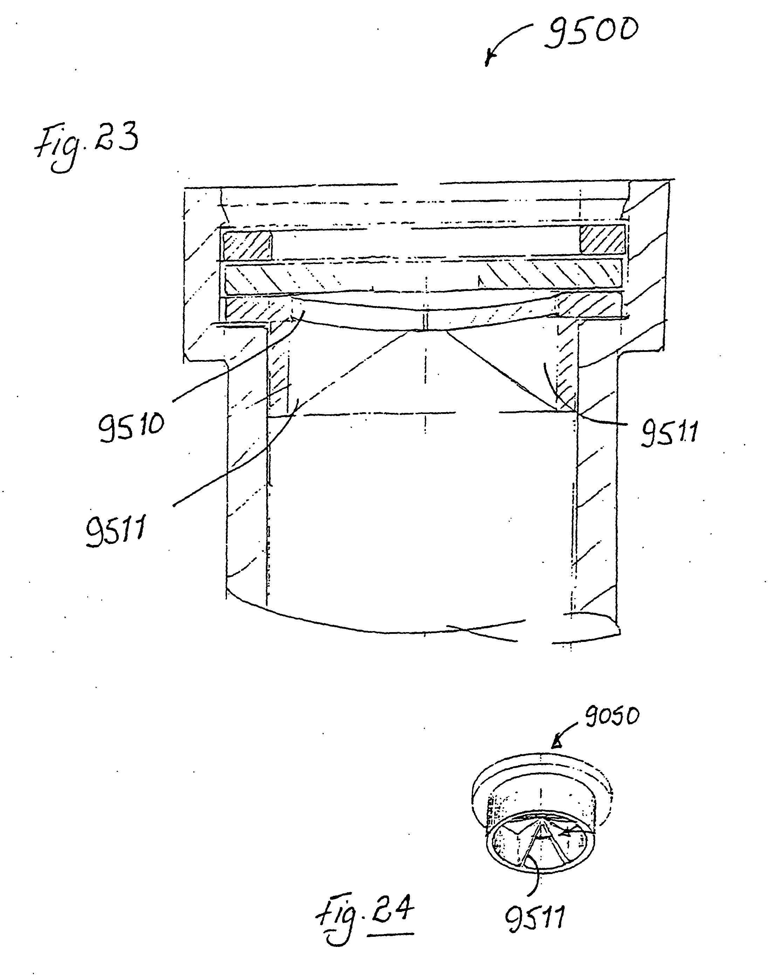

Figs. 23 and 24 show a similar seal to that illustrated in Figs. 21 and 22 illustrated generally by the reference numeral 9500. In this seal 9500 the slits 9050 arc orthogonally directed as before on a concave disc 9510. The concave nature of the disc 9510 ensures that the slits 9050 are pressed together. Upward movement beyond a resting position of any portion of the disc 9510 is prevented by the provision of four webs 9511 on the underside of the disc. 9510 intermediate the slits 9050.

It will be noted that the invention is directed to wards providing a reduced trauma, low profile, airtight cannula. It will be appreciated also that the device is particularly suited for use with a trocar and is ideal for applications in which the device is positioned from within the body cavity. Thus, the invention provides a simple method and device which prevents accidental damage to the patients tissue or organs by ensuring that the cutting or piercing point of the trocar is directed away from the patient's body during insertion.

It will be understood that the cutting or piercing method may optionally include the use of an energy source such as a heater or spring-loaded mechanism for executing the cut using a minimum of force.

To avoid unnecessarily obscuring the present invention, detailed description of the valve has not been included, as it will be apparent to those skilled in the art that a wide variety of valves may be successfully employed. One such possibility is a twist valve, but any suitable valve may be used.

It will be noted that the external ring may define a circular entry port or a partial funnel with an oversized opening to facilitate threading of components into the cannula.

It will also be noted that the internal and external rings may be suitably coated with airtight, antiseptic and or shock absorbent material to prevent trauma to the abdominal wall and loss of gas from the body cavity.

It will be understood that the anchor ring may be extended to incorporate a valve, which allows insertion of instruments thereby reducing the weight of the device.

It will also be understood that the device may incorporate a detachable security retainer formed for engagement with a surgeon's hand or instrument to prevent loss of the device in the cavity prior to insertion.

It will further be understood that the device may incorporate an adjustable pressure release valve. This will allow the surgeon to inflate the body cavity to a desired level and ensure a constant level of body cavity inflation by continuously supplying inflation gas and venting any excess through the pressure release valve. This prevents excessive movement of the cavity wall during the procedure, which can inconvenience the surgeon and cause trauma to the patient

It will be noted that the devices described herein may be provided as modular devices and that the device may be formed to accommodate different sizes of seals and valves to allow the device a broad scope of application.

It will also be noted that the device may optionally include an insufflation lumen or conduit to provide pneumoperitoneum at the operating site.

It will also be understood that the component parts of the device may be transparent to facilitate a surgeon during the course of an operation.

It will of course be understood that the invention is not limited to the specific details described herein, which are given by way of example only, and that various modifications and alterations are possible within the scope of the invention as defined in the appended claims.

## Claims

1. A surgical device (1) for use in minimally invasive surgery of the type where a body cavity is inflated to be accessible to a surgeon through an access port surrounding an incision in a patient's body, the device (1) being formed to allow insertion of medical equipment and comprising: -
a cannula (6) defining a conduit into the body cavity;
a trocar (5) carried on the cannula (6) and formed for piercing or cutting tissue to position the cannula (6); and
fixing means (7) for removably securing the cannula (6) imposition on the patient during surgery,
**characterised in that** the trocar (5) is removably mounted on the cannula (6), with the trocar (5) providing a gas-tight cap for the cannula when the trocar is mounted on the cannula thereby enabling the trocar (5) to be inserted into the body cavity through the access port and to cut or pierce tissue outwardly from within the body cavity out to an operating site, with no escape of gas from the body cavity.

2. A surgical device as claimed in claim 1, wherein the trocar (5) and cannula (6) are removably mounted with complementary engageable short threads (12).

3. A surgical device (1) as claimed in claim 1 or claim 2, wherein the trocar (5) is provided with an integral cutting element (8), thereby facilitating the cutting of the body cavity wall (2) to allow introduction of the cannula (6).

4. A surgical device (1) as claimed in claim 3, wherein the trocar (5) incorporates an extension shoulder (9).

5. A surgical device (1) as claimed in any preceding claim, wherein the trocar (5) incorporates guard means for preventing injury to the surgeon.

6. A surgical device (1) as claimed in any preceding claim, wherein the cannula (6) incorporates means (15) for releaseably attaching the cannula to an interior (3) of the body cavity.

7. A surgical device as claimed in claim 6, wherein the means (15) for attaching the cannula (6) to an interior (3) of the body cavity is provided by an internal distal ring(15).

8. A surgical device (1) as claimed in claim 7, in which the internal distal ring (35) and the cannula (6) are integrally formed as a single unit.

9. A surgical device (1) as claimed in any preceding claim; wherein the cannula (6) incorporates a valve to prevent loss of gas from the body cavity when the canula is in position.

10. A surgical device as claimed in any preceding claim, wherein the fixing means incorporates an anchor ring (7) formed for releasable engagement with a proximal end of the cannula (6) extending from the body when the cannula (6) is in position in the body cavity.

11. A surgical device as claimed in claim 10, wherein the anchor ring (7) incorporates a thread for engaging on the same thread (12) used to secure the trocar (5) prior to installation of the cannula (6).

12. A surgical device as claimed in claim 10 or 11, wherein the anchor ring (7) incorporates a valve.

13. A surgical device as claimed in any preceding claim, incorporating an external seal (901) and an internal valve (902), the seal (901) and valve (902) being mounted about opposing ends of the cannula (6).

14. A surgical device as claimed in claim 13, wherein the cannula (6) is integrally formed with the seal (901) and valve (902).

15. A surgical device as claimed in claim 13 or 14, wherein the valve (902) is integrally formed at one end of the cannula (6) and has means (905) for releaseably engaging a seal housing (901) at an opposing end.

16. A surgical device as claimed in claim 15, in which the.seal housing (901) incorporates a diaphragm seal.

17. A surgical device as claimed in any of claims 13 to 16; wherein the seal housing (901) defines an extended entry port (904).

18. A surgical device as claimed in claim 17, wherein the entry.port (904) has a conical . section.

19. A surgical device as claimed in any preceding claim, wherein the cannula (6) incorporates an insufflation port (910).

20. A surgical device as claimed in claim 19, in which the insufflation port (910) communicates with an insufflation lumen (912) having insulation ducts (911) communicating between the port (910) and the body cavity.

21. A surgical device as claimed in claim 20, wherein the insufflation lumen (912) is carried on an exterior surface of the cannula (6).

22. A surgical device as claimed in any of claims 11 to 21, wherein the anchor ring (7) incorporates cushion means to prevent trauma to the body cavity wall and ensure a gas tight seal.

23. A surgical device as claimed in any of claims 11 to 22; wherein the distal ring (15) incorporates cushion means to prevent trauma to the body cavity wall and ensure a gas tight seal.

24. A surgical device as claimed in any preceding claim, incorporating a detachable security retainer formed for engagement with a surgeon's hand or instrument to prevent loss of the device in the cavity prior to being fixed in position on a patient.

25. A surgical device as claimed in any preceding claim, incorporating an adjustable pressure release valve.

## Patentansprüche

1. Eine chirurgische Vorrichtung (1) zur Anwendung in minimal invasiver Chirurgie des Typs, wobei eine Körperhöhle aufgeblasen wird, um für einen Chirurgen durch eine Zugangsöffnung, die einen Einschnitt in einem Körper eines Patienten umgibt, zugänglich zu sein, wobei die Vorrichtung (1) so geformt ist, dass sie das Einbringen medizinischer Ausrüstung gestattet, und folgendes umfasst:
eine Kanüle (6), die eine Leitung in die Körperhöhle definiert;
einen Trokar (5), der auf der Kanüle (6) getragen wird und zum Durchstechen oder Durchschneiden von Gewebe geformt ist, um die Kanüle (6) zu positionieren; und
Befestigungsmittel (7) zum lösbaren Sichern der Kanüle (6) in Position an dem Patienten während des chirurgischen Eingriffs,
**dadurch gekennzeichnet, dass** der Trokar (5) abnehmbar an der Kanüle (6) montiert ist, wobei der Trokar (5) eine gasdichte Verschlusskappe für die Kanüle verschafft, wenn der Trokar an der Kanüle montiert ist, wodurch das Einführen des Trokars (5) in die Körperhöhle durch die Zugangsöffnung und das Durchschneiden oder Durchstechen von Gewebe nach außen von innerhalb der Körperhöhle nach außen zu einer Operationsstelle ermöglicht wird, ohne dass Gas aus der Körperhöhle entweicht.

2. Eine chirurgische Vorrichtung, wie in Anspruch 1 beansprucht, wobei der Trokar (5) und die Kanüle (6) lösbar montiert sind, mit komplementären kurzen Gewinden (12), die miteinander in Eingriff gebracht werden können.

3. Eine chirurgische Vorrichtung (1), wie in Anspruch 1 oder Anspruch 2 beansprucht, wobei der Trokar (5) mit einem integralen Schneidelement (8) versehen ist, wodurch das Durchschneiden der Wand der Körperhöhle (2), um das Einführen der Kanüle (6) zuzulassen, erleichtert wird.

4. Eine chirurgische Vorrichtung (1), wie in Anspruch 3 beansprucht, wobei der Trokar (5) einen Schulterfortsatz (9) umfasst.

5. Eine chirurgische Vorrichtung (1), wie in einem der vorgenannten Ansprüche beansprucht, wobei der Trokar (5) Absicherungsmittel zur Verhinderung einer Verletzung des Chirurgen umfasst.

6. Eine chirurgische Vorrichtung (1), wie in einem der vorgenannten Ansprüche beansprucht, wobei die Kanüle (6) Mittel (15) zum lösbaren Befestigen der Kanüle an einem Inneren (3) der Körperhöhle umfasst.

7. Eine chirurgische Vorrichtung, wie in Anspruch 6 beansprucht, wobei das Mittel (15) zum Befestigen der Kanüle (6) an einem Inneren (3) der Körperhöhle durch einen internen distalen Ring (15) verschafft wird.

8. Eine chirurgische Vorrichtung (1), wie in Anspruch 7 beansprucht, wobei der interne distale Ring (15) und die Kanüle (6) integral als eine einzige Einheit ausgebildet sind.

9. Eine chirurgische Vorrichtung (1), wie in einem der vorgenannten Ansprüche beansprucht, wobei die Kanüle (6) ein Ventil umfasst, um Gasverlust aus der Körperhöhle zu verhindern, wenn die Kanüle in Position ist.

10. Eine chirurgische Vorrichtung, wie in einem der vorgenannten Ansprüche beansprucht, wobei das Befestigungsmittel einen Verankerungsring (7) umfasst, der zum lösbaren Eingriff mit einem proximalen Ende der Kanüle (6), das aus dem Körper ragt, wenn die Kanüle (6) in Position in der Körperhöhle ist, ausgebildet ist.

11. Eine chirurgische Vorrichtung, wie in Anspruch 10 beansprucht, wobei der Verankerungsring (7) ein Gewinde zum Angreifen an demselben, zur Sicherung des Trokars (5) vor dem Installieren der Kanüle (6) verwendeten Gewinde (12) umfasst.

12. Eine chirurgische Vorrichtung, wie in Anspruch 10 oder 11 beansprucht, wobei der Verankerungsring (7) ein Ventil umfasst.

13. Eine chirurgische Vorrichtung, wie in einem der vorgenannten Ansprüche beansprucht, welche eine externe Abdichtung (901) und ein internes Ventil (902) umfasst, wobei Abdichtung (901) und Ventil (902) über entgegengesetzte Enden der Kanüle (6) montiert sind.

14. Eine chirurgische Vorrichtung, wie in Anspruch 13 beansprucht, wobei die Kanüle (6) integral mit der Abdichtung (901) und dem Ventil (902) geformt ist.

15. Eine chirurgische Vorrichtung, wie in Anspruch 13 oder 14 beansprucht, wobei das Ventil (902) integral an einem Ende der Kanüle (6) geformt ist und Mittel (905) zum lösbaren Angreifen an einem Dichtungsgehäuse (901) an einem entgegengesetzten Ende aufweist.

16. Eine chirurgische Vorrichtung, wie in Anspruch 15 beansprucht, wobei das Dichtungsgehäuse (901) eine Membrandichtung umfasst.

17. Eine chirurgische Vorrichtung, wie in einem der Ansprüche 13 bis 16 beansprucht, wobei das Dichtungsgehäuse (901) eine verlängerte Eintrittsöffnung (904) definiert.

18. Eine chirurgische Vorrichtung, wie in Anspruch 17 beansprucht, wobei die Eintrittsöffnung (904) einen konischen Querschnitt hat.

19. Eine chirurgische Vorrichtung, wie in einem der vorgenannten Ansprüche beansprucht, wobei die Kanüle (6) eine Aufblaseöffnung (910) umfasst.

20. Eine chirurgische Vorrichtung, wie in Anspruch 19 beansprucht, wobei die Aufblaseöffnung (910) mit einem Aufblaseinnenraum (912) kommuniziert, der Aufblasekanäle (911) aufweist, die zwischen der Öffnung (910) und der Körperhöhle kommunizieren.

21. Eine chirurgische Vorrichtung, wie in Anspruch 20 beansprucht, wobei der Aufblaseinnenraum (912) auf einer Außenfläche der Kanüle (6) getragen wird.

22. Eine chirurgische Vorrichtung, wie in einem der Ansprüche 11 bis 21 beansprucht, wobei der Verankerungsring (7) Polstermittel umfasst, um ein Trauma der Körperhöhlenwand zu verhindern und eine gasdichte Abdichtung sicherzustellen.

23. Eine chirurgische Vorrichtung, wie in einem der Ansprüche 11 bis 22 beansprucht, wobei der distale Ring (15) Polstermittel umfasst, um ein Trauma der Körperhöhlenwand zu verhindern und eine gasdichte Abdichtung sicherzustellen.

24. Eine chirurgische Vorrichtung, wie in jedem vorgenannten Anspruch beansprucht, die eine abnehmbare Sicherheitshalterung umfasst, die zum Eingriff an der Hand oder dem Instrument eines Chirurgen geformt ist, um ein Verlieren der Vorrichtung in der Höhlung zu verhindern, bevor sie in Position an einem Patienten befestigt wird.

25. Eine chirurgische Vorrichtung, wie in einem der vorgenannten Ansprüche beansprucht, welche ein einstellbares Druckregelventil umfasst.

## Revendications

1. Dispositif chirurgical (1) à utiliser dans la chirurgie la moins invasive possible, du type dans lequel une cavité corporelle est gonflée pour être accessible à un chirurgien à travers un orifice d'accès entourant une incision pratiquée dans le corps d'un patient, le dispositif (1) étant réalisé pour permettre l'insertion d'un équipement médical et comprenant :
une canule (6) définissant un conduit dans la cavité corporelle ;
un trocart (5) supporté par la canule (6) et réalisé pour percer ou découper un tissu dans le but de positionner la canule (6) ; et
un moyen de fixation (7) pour fixer de manière amovible la canule (6) en position au patient lors d'une opération chirurgicale ;
**caractérisé en ce que** le trocart (5) est monté de manière amovible sur la canule (6), le trocart (5) procurant un chapeau étanche aux gaz pour la canule, lorsque le trocart est monté sur la canule, pour permettre l'insertion du trocart (5) dans la cavité corporelle via l'orifice d'accès et pour découper ou pour percer le tissu vers l'extérieur depuis la cavité corporelle jusqu'à un site opératoire, en l'absence d'échappement de gaz à partir de la cavité corporelle.

2. Dispositif chirurgical selon la revendication 1, dans lequel le trocart (5) et la canule (6) sont montés de manière amovible avec des filets de vis courts (12) aptes à s'engrener de manière complémentaire.

3. Dispositif chirurgical (1) selon la revendication 1 ou 2, dans lequel le trocart (5) est muni d'un élément de découpe solidaire (8), pour faciliter la découpe de la paroi de la cavité corporelle (2) dans le but de permettre l'introduction de la canule (6).

4. Dispositif chirurgical (1) selon la revendication 3, dans lequel le trocart (5) englobe un épaulement de prolongement (9).

5. Dispositif chirurgical (1) selon l'une quelconque des revendications précédentes, dans lequel le trocart (5) englobe un moyen de protection pour empêcher le chirurgien de se blesser.

6. Dispositif chirurgical (1) selon l'une quelconque des revendications précédentes, dans lequel la canule (6) englobe un moyen (15) pour la fixation amovible de la canule dans l'espace interne (3) de la cavité corporelle.

7. Dispositif chirurgical selon la revendication 6, dans lequel le moyen (15) pour la fixation de la canule (6) dans l'espace interne (3) de la cavité corporelle est fourni par un anneau distal interne (15).

8. Dispositif chirurgical (1) selon la revendication 7, dans lequel l'anneau distal interne (15) et la canule (6) sont réalisés en une seule pièce pour former une unité individuelle.

9. Dispositif chirurgical selon l'une quelconque des revendications précédentes, dans lequel la canule (6) englobe un clapet pour empêcher la perte de gaz à partir de la cavité corporelle lorsque la canule se trouve en position.

10. Dispositif chirurgical selon l'une quelconque des revendications précédentes, dans lequel le moyen de fixation englobe un anneau d'ancrage (7) réalisé pour obtenir un engrènement amovible avec l'extrémité proximale de la canule (6) s'étendant à partir du corps lorsque la canule (6) se trouve en position dans la cavité corporelle.

11. Dispositif chirurgical selon la revendication 10, dans lequel l'anneau d'ancrage (7) englobe un filet de vis à des fins d'engrènement avec le même filet de vis (12) que celui utilisé pour fixer le trocart (5) avant le montage de la canule (6).

12. Dispositif chirurgical selon la revendication 10 ou 11, dans lequel l'anneau d'ancrage (7) englobe un clapet.

13. Dispositif chirurgical selon l'une quelconque des revendications précédentes, englobant un joint d'étanchéité externe (901) et un clapet interne (902), le joint d'étanchéité (901) et le clapet (902) étant montés autour des extrémités opposées de la canule (6).

14. Dispositif chirurgical selon la revendication 13, dans lequel la canule est réalisée en une seule pièce avec le joint d'étanchéité (901) et le clapet (902).

15. Dispositif chirurgical selon la revendication 13 ou 14, dans lequel le clapet (902) est réalisé en une seule pièce avec une extrémité de la canule (6) et possède un moyen (905) pour l'engrènement amovible d'un logement de joint d'étanchéité (901) à l'extrémité opposée.

16. Dispositif chirurgical selon la revendication 15, dans lequel le logement de joint d'étanchéité (901) englobe un joint d'étanchéité du type à diaphragme.

17. Dispositif chirurgical selon l'une quelconque des revendications 13 à 16, dans lequel le logement de joint d'étanchéité (901) définit un orifice d'entrée étendu (904).

18. Dispositif chirurgical selon la revendication 17, dans lequel l'orifice d'entrée (904) possède une section conique.

19. Dispositif chirurgical selon l'une quelconque des revendications précédentes, dans lequel la canule (6) englobe un orifice d'insufflation (910).

20. Dispositif chirurgical selon la revendication 19, dans lequel l'orifice d'insufflation (910) communique avec une lumière d'insufflation (912) possédant des conduits d'insufflation (911) établissant une communication entre l'orifice (910) et la cavité corporelle.

21. Dispositif chirurgical selon la revendication 20, dans lequel la lumière d'insufflation (912) est supportée sur la surface externe de la canule (6).

22. Dispositif chirurgical selon l'une quelconque des revendications 11 à 21, dans lequel l'anneau d'ancrage (7) englobe un moyen de rembourrage pour empêcher un traumatisme de la paroi de la cavité corporelle et pour garantir un joint étanche aux gaz.

23. Dispositif chirurgical selon l'une quelconque des revendications 11 à 22, dans lequel l'anneau distal (15) englobe un moyen de rembourrage pour empêcher un traumatisme de la paroi de la cavité corporelle et pour garantir un joint étanche aux gaz.

24. Dispositif chirurgical selon l'une quelconque des revendications précédentes, englobant un dispositif de retenue de sécurité amovible réalisé à des fins de mise en contact avec un instrument ou avec la main du chirurgien pour empêcher la perte du dispositif dans la cavité, avant la fixation de ce dernier en position sur un patient.

25. Dispositif chirurgical selon l'une quelconque des revendications précédentes, englobant un clapet de décharge réglable.
